Europäisches Patentamt

(19) European Patent Office

Office européen des brevets

(11) Veröffentlichungsnummer: **0 205 785**
**B1**

(12) **EUROPÄISCHE PATENTSCHRIFT**

(45) Veröffentlichungstag der Patentschrift:
07.02.90

(21) Anmeldenummer: 86104669.6

(22) Anmeldetag: 05.04.86

(51) Int. Cl.⁵: **A 61 F 2/64**

(54) Arretierbares Kniegelenk.

(30) Priorität: 28.05.85 DE 8515598 U

(43) Veröffentlichungstag der Anmeldung:
30.12.86 Patentblatt 86/52

(45) Bekanntmachung des Hinweises auf die Patenterteilung:
07.02.90 Patentblatt 90/06

(84) Benannte Vertragsstaaten:
DE FR GB IT SE

(56) Entgegenhaltungen:
EP-A-0 068 658
DE-U-8 515 598
FR-A-490 619
FR-A-547 292
FR-A-633 838
GB-A-2 080 114

(73) Patentinhaber: Otto Bock Orthopädische Industrie Besitz-
und Verwaltungs-Kommanditgesellschaft
Industriestrasse
D-3408 Duderstadt 1 (DE)

(72) Erfinder: Haupt, Werner
Friedensstrasse 39
D-3408 Duderstadt (DE)

(74) Vertreter: Lins, Edgar, Dipl.-Phys.
Patentanwälte Gramm + Lins Theodor-Heuss-Strasse 2
D-3300 Braunschweig (DE)

EP 0 205 785 B1

## Beschreibung

Die Erfindung betrifft ein Kniegelenk mit einem an einem Oberschenkelteil angelenkten und in der gestreckten Stellung des Kniegelenks arretierten Unterschenkelteil, dessen Arretierungsvorrichtung im Unterschenkelteil angeordnet ist und mit einem über die Kontur des Unterschenkelteils nach oben herausragenden Verriegelungselement in eine Ausnehmung des Oberschenkelteils eingreift, wobei die Arretierung mittels eines in das Unterschenkelteil eingeführten Bowdenzuges über eine im Unterschenkelteil gelagerte Wippe lösbar ist, deren erster Arm die Stellung des Verriegelungselements bestimmt und deren zweiter Arm mit dem Bowdenzug verbunden ist.

Arretierbare Kniegelenke dienen dazu, dem Prothesenträger ein sicheres Gehgefühl mit dem steifgestellten Kniegelenk zu ermöglichen und ihn von der Gefahr eines unbeabsichtigten Einknickens des Kniegelenks zu entlasten. Selbstverständlich ist es erforderlich, eine lösbare Arretierung vorzusehen, damit das Einknicken des Kniegelenkes zum Sitzen o. ä. möglich ist. Üblicherweise befinden sich die Arretierungsmittel und der Auslösemechanismus im Oberschenkelteil der Prothese. Aus der GB-A-2 080 114 ist es bekannt, als Verriegelungselement einen federbelasteten Bolzen zu verwenden, der über die untere Kontur des Oberschenkelteils durch Federn herausdrückbar ist, in eine entsprechende Ausnehmung des Unterschenkelteils eingreift und die Arretierung besorgt. Die Rückstellung erfolgt durch ein Zurückziehen des Bolzens gegen die Federkraft. Diese übliche Anbringung des Arretierungsmechanismus im Oberschenkelteil führt zu kosmetischen Schwierigkeiten und zu Problemen bei der Bedienbarkeit der Entarretierung und ist bei langen Oberschenkelstümpfen nicht anwendbar.

Aus der FR-A-633 838 ist ein Kniegelenk der eingangs erwähnten Art bekannt, bei dem also die Arretierungsvorrichtung im Unterschenkelteil angeordnet ist. Die Entarretierung bedingt einen Zug an dem in das Oberschenkelteil ragenden Verriegelungselement nach unten. Um eine starke Knickung des Entarretierungsseils zu vermeiden, ist eine Wippe vorgesehen, so daß die Entarretierung durch einen Zug an dem Seil nach oben erfolgen kann. Durch eine am Unterschenkelteil befestigte Spiralfeder wird der Arm der Wippe, an dem das Entarretierungsseil angreift, nach unten gedrückt, so daß das Verriegelungselement durch die Feder in seine Arretierungsstellung gedrückt wird.

Allen bekannten arretierbaren Kniegelenken ist gemein, daß ihre Konstruktion auf die Arretierungsvorrichtung speziell abgestellt ist. Die verschiedenen Teile der Arretierungsvorrichtung werden in unterschiedlichen Einrichtungen, beispielsweise des Unterschenkelteils gehalten, so daß das Unterschenkelteil mit diesen verschiedenen Einrichtungen versehen sein muß und die Teile der Arretierungsvorrichtung separat montiert werden müssen. Die Teile müssen zueinander justiert werden. Der hierfür erforderliche Aufwand ist groß und die Funktionssicherheit nicht optimal.

Der Erfindung liegt die Aufgabe zugrunde, ein Kniegelenk der eingangs erwähnten Art zu erstellen, das einfach aufbaubar und funktionssicher ist.

Diese Aufgabe wird bei einem Kniegelenk der eingangs erwähnten Art erfindungsgemäß dadurch gelöst, daß ein in das Unterschenkelteil eingesetzter Montageblock sowohl einen als Verriegelungselement dienenden Bolzen, der mittels einer in dem Montageblock gehaltenen Feder in die Arretierungsstellung gedrückt wird, führt und hält, als auch die Wippe an seiner Unterseite trägt, deren erster Arm mit dem unteren Ende des Bolzens verbunden ist.

Die erfindungsgemäße Verriegelungsvorrichtung ist komplett im dem Montageblock untergebracht. Die Halterung und Führung der Teile der Verriegelungsvorrichtung erfolgt komplett in dem Montageblock, so daß sich ein einfacher und kompakter Aufbau ergibt.

In einer besonders bevorzugten Ausführungsform sind der Montageblock und die Wippe durch ein Wippgelenk miteinander verbunden, das ausschließlich durch eine Ausnehmung-Steg-Ausnehmung- bzw. Steg-Ausnehmung-Steg-Konfiguration der aneinander liegenden Seiten von Montageblock und Wippe gebildet ist. Durch die verzahnte Ausbildung der aneinander liegenden Seiten von Montageblock und Wippe ist ohne zusätzliche Befestigungsmittel im zusammengebauten Zustand sichergestellt, daß nur die zum Kippen benötigte Rotationsbewegung zwischen Montageblock und Wippe möglich ist. Im übrigen wird die Wippe an dem Montageblock durch den federbelasteten Kolben einerseits und den Bowdenzug andererseits gehalten.

Vorzugsweise weist der Montageblock einen über den zweiten Arm der Wippe ragenden Ansatz mit einem Führungsloch für den Bowdenzug auf. Dadurch wird der Bowdenzug durch die beiden, vozugsweise aus Kunststoff hergestellten Teile Montageblock und Wippe definiert geführt.

Der Arretierungsmechanismus läßt sich sowohl rechts wie auch links verwenden, wenn sich der Ansatz des Montageblocks und der zweite Arm der Wippe gabelförmig und symmetrisch zur senkrecht auf der Wippachse stehenden Mittelebene des Montageblocks erstrecken. In diesem Fall kann der Bowdenzug auf beiden Seiten geführt und gehalten werden.

Wenn dann das freie Ende des Bowdenzugs an einem kreisförmig ausgebildeten, mit einem Auslösehebel versehenen Auslöseteil anliegt und befestigt ist, das drehbar in einem am Oberschenkelteil befestigten Gehäuse gelagert ist, ist das Auslöseteil in entsprechender Weise an den Montageblock und die Wippe bezüglich der Rechts-Links-Montage angepaßt. Hierzu ist es zweckmäßig, wenn das Gehäuse zwei Einlaßführungen für den Bowdenzug aufweist, die etwa tangential auf der Seite des Auslösehebels und auf der gegenüberliegenden Seite auf das Auslöseteil gerichtet sind. Je nach der Anbringung auf der rechten oder linken Seite wird der Bowdenzug in der einen oder anderen Richtung um das Auslöseteil geschlungen. Die Umschlingungsrichtung läßt auch zu, daß der Auslösehebel zur Entarretierung - je nach Wunsch - entweder nach unten gedrückt oder nach oben gezogen wird.

Somit läßt sich unter Verwendung derselben Teile der Arretierungsmechanismus sowohl für Rechts-als auch für Linksbetätigung nach Wunsch des Prothesenträgers montieren als auch dem Wunsch des Prothesenträgers Rechnung tragen, die Entarretierung durch einen Zug nach oben oder einen Druck nach unten zu bewirken.

Die Erfindung soll im folgenden anhand eines in der i Zeichnung dargestellten Ausführungsbeispiels näher erläutert werden. Es zeigen:

Figur 1: einen Längsschnitt durch ein arretiertes Kniegelenk;
Figur 2: ein Auslöseteil mit abgenommener Kappe des Gehäuses.

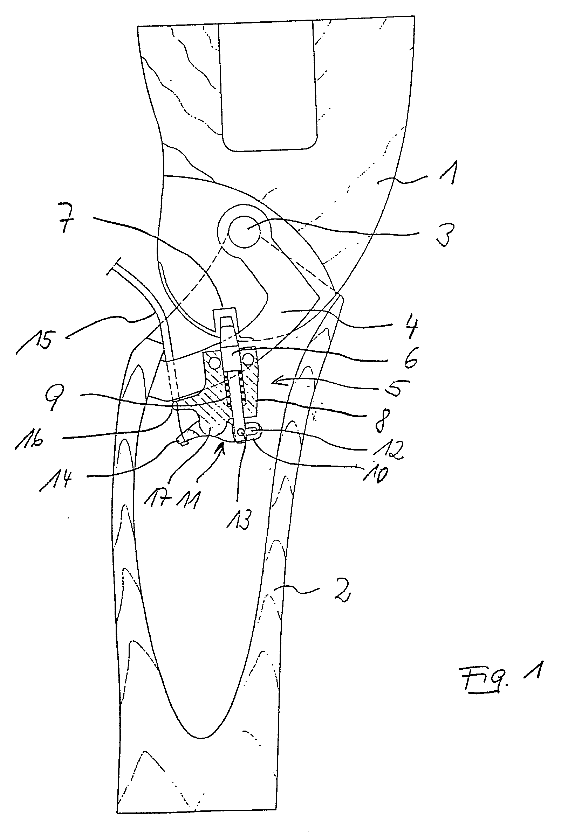

Das in Figur 1 dargestellte Kniegelenk besteht aus einem Oberschenkelteil 1 und einem Unterschenkelteil 2, die in dem dargestellten Ausführungsbeispiel beide aus Holz gefertigt sind. Das Oberschenkelteil 1 trägt eine Drehachse 3, an der das Unterschenkelteil 2 mittels an ihm befestigter, im wesentlichen L-förmiger Lenker 4 angelenkt ist.

Das Unterschenkelteil 2 trägt eine Arretierungsvorrichtung 5, von der ein oberes Ende eines Bolzens 6 über die obere Kontur des Unterschenkelteils 2 hinausragt und in eine zylindrische Ausnehmung 7 auf der Unterseite des Oberschenkelteils 1 eingreift, wenn sich das Kniegelenk in der gestreckten Stellung befindet. Der Bolzen 6 ist in einem Montageblock 8 geführt und wird von einer in dem Montageblock 8 gehaltenen Schraubenfeder 9 in die Arretierungsstellung gedrückt. Das zweite Ende des Bolzens 6 ragt aus dem Montageblock 8 hinaus und ist an einem ersten Arm 10 einer Wippe 11 befestigt. Der erste Art 10 der Wippe 11 umschließt das Ende des Bolzens 6 und weist auf zwei gegenüberliegenden Seiten Langlöcher 12 auf, durch die ein das Ende des Bolzens 6 durchdringender Splint 13 ragt.

Ein zweiter Arm 14 der Wippe 11 dient zur Befestigung eines Bowdenzugs 15, der in das Unterschenkelteil 2 eingeführt ist. Zur Führung des Bowdenzugs 15 weist der Montageblock einen über den zweiten Arm 14 der Wippe 11 ragenden Ansatz 16 auf, der mit einem entsprechenden Führungsloch für den Bowdenzug 15 versehen ist und zur Abstützung der Bowdenzugspirale dient.

Der Ansatz 16 und der zweite Arm 14 sind gabelförmig ausgebildet und erstrecken sich symmetrisch zu der Mittelebene des Montageblocks 8, die der Zeichenebene entspricht und senkrecht zur Kippachse der Wippe 11 steht.

Die Wippe 11 ist an dem Montageblock 8 mittels einer (nicht dargestellten) Ausnehmung gelagert, die beidseitig von Stegen begrenzt ist und eine ballige Auswölbung 17 des Montageblocks 8 aufnimmt, die seitlich von Ausnehmungen für die Stege der Wippe 11 begrenzt ist. Diese verzahnte Anordnung von Wippe 11 und Montageblock 8 bildet ein Kipplager für die Wippe 11, das keine weitere Befestigungen erfordert, da die Wippe 11 durch den Bowdenzug 15 und den Bolzen 6 gegen ein Abfallen nach unten von dem Montageblock 8 gesichert ist.

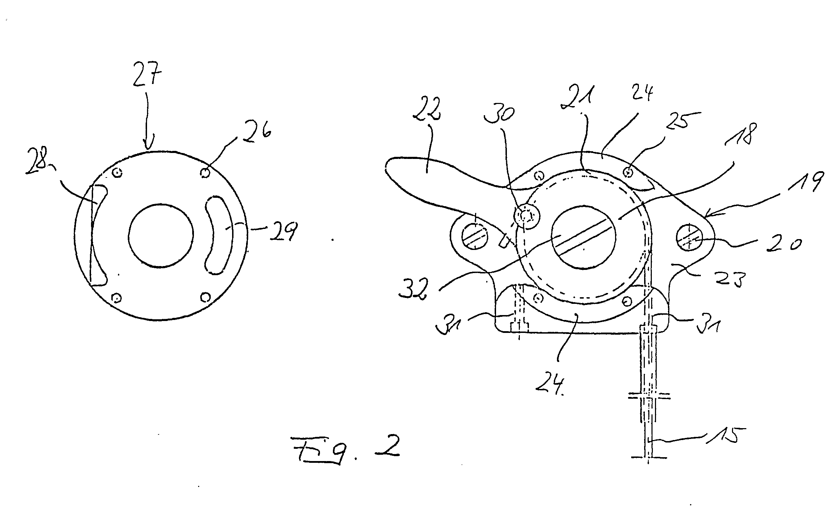

Figur 2 zeigt ein zugehöriges Auslöseteil 18, das in einem Gehäuse 19 gelagert ist, das seinerseits fest am Oberschenkelteil 1 mittels Schrauben 20 befestigt ist. Das Auslöseteil 18 ist kreisförmig ausgebildet und weist eine umlaufende Nut 21 zur Führung des Bowdenzugs 15 auf. An das Auslöseteil 18 ist ein Auslösehebel 22 angeformt.

Das Gehäuse 19 weist eine rückwärtige Trageplatte 23 auf, von der sich oben und unten das kreisförmige Auslöseteil 18 begrenzende, eine teilkreisförmige Innenkontur aufweisende Stege 24 erheben. Die Stege 24 weisen Sacklöcher 25 auf, in die Stifte 26 eines Gehäusedeckels 27 einsteckbar sind, mit dem das Gehäuse verschließbar ist. Der Gehäusedeckel 27 weist einen zwischen die Stege 24 auf der dem Auslösehebel 22 abgewandten Seite passenden, Stegabschnitt 28 auf, der die Lagerung des Auslöseteils 18 unterstützt. Auf der gegenüberliegenden Seite ist eine kreisabschnittförmige Schlitzführung 29 im Deckelteil vorgesehen, in die ein Befestigungsbolzen 30 für das freie Ende des Bowdenzugs 15 hineinragen kann.

Es ist ersichtlich, daß das Auslöseteil 18 in der in Figur 2 dargestellten Stellung mit dem Auslösehebel 22 nach links, aber auch in einer Stellung mit dem Auslösehebel 22 nach rechts einsetzbar ist. Um in beiden Fällen beispielsweise durch Drücken des Hebels 22 die Arretierung über den Bowdenzug 15 zu lösen, muß der Bowdenzug in die andere der beiden Einlaßführungen 31 in dem unteren Steg 24 des Gehäuses 19 eingeführt werden. Die beiden Einlaßführungen 31 sind so positioniert, daß der durch sie hindurchgeführte Bowdenzug 15 tangential auf verschiedenen Seiten des kreisförmigen Auslöseteils 18 auf dieses gerichtet ist. Die Einlaßführungen 31 weisen einen Absatz auf, der zur Abstützung dieses Endes der Bowdenzugspirale dient.

Figur 2 zeigt eine etwa halbkreisförmige Umschlingung des Bowdenzugs 15 um das Auslöseteil 18. Der Bowdenzug 15 wird durch Drücken des Auslösehebels 22 nach unten angezogen, wodurch die Wippe 11 den Bolzen 6 aus der Ausnehmung 7 herauszieht und somit die Arretierung löst.

Wird der Bowdenzug bei derselben Anordnung durch die andere Einlaßführung 31 geführt und mittels des Bolzens 30 befestigt, führt ersichtlich ein Anheben des Auslösehebels 22 zum Anziehen des Bowdenzugs 15 und somit zur Entarretierung des Kniegelenkes. Die Anordnung der beiden Einlaßführungen 31 ermöglicht daher nicht nur die Wahl der Recht-Links-Montage der Entarretierungsanordnung, sondern auch die Wahl des Prothesenträgers, die Entarretierung durch ein Nach-untenDrücken oder durch ein Nach-oben-Ziehen des Auslösehebels 22 zu bewirken. Das Auslöseteil 18 ist drehbar mit dem Gehäuse 19 über eine Schraube 32 verbunden, so daß das Herabdrücken bzw. Heraufziehen des Auslösehebels 22 auf einem Kreisbahnabschnitt geschieht.

Der Montageblock 8 und die Wippe 11 können als leichte Kunststoffteile ausgebildet sein. Auch das Oberschenkelteil 1 und das Unterschenkelteil 2 können aus anderen Materialien als Holz, beispielsweise Kunststoff, bestehen.

## Patentansprüche

1. Kniegelenk mit einem an einem Oberschenkelteil (1) angelenkten und in der gestreckten Stellung des Kniegelenks arretierten Unterschenkelteil (2), dessen Arretierungsvorrichtung (5) im Unterschenkelteil (2) angeordnet ist und mit einem über die Kontur des Unterschenkelteils (2) nach oben herausragenden Verriegelungselement (6) in eine Ausnehmung (7) des Oberschenkelteils (1) eingreift, wobei die Arretierung mittels eines in das Unterschenkelteil (2) eingeführten Bowdenzuges (15) über eine im Unterschenkelteil (2) gelagerte Wippe (11) lösbar ist, deren erster Arm (10) die Stellung des Verriegelungselements (6) bestimmt und deren zweiter Arm (14) mit dem Bowdenzug verbunden ist, dadurch gekennzeichnet, daß ein in das Unterschenkelteil (2) eingesetzter Montageblock (8) sowohl einen als Verriegelungselement dienenden Bolzen (6), der mittels einer in dem Montageblock gehaltenen Feder (9) in die Arretierungsstellung gedrückt wird, führt und hält, als auch die Wippe (11) an seiner Unterseite trägt, deren erster Arm (10) mit dem unteren Ende des Bolzens (6) verbunden ist.

2. Kniegelenk nach Anspruch 1, dadurch gekennzeichnet, daß der Montageblock (8) und die Wippe (11) durch ein Wippgelenk (17) miteinander verbunden sind, das ausschließlich durch eine Ausnehmung-Steg-Ausnehmung bzw. Steg-Ausnehmung-Steg-Konfiguration der aneinanderliegenden Seiten vom Montageblock (8) und Wippe (11) gebildet ist.

3. Kniegelenk nach Anspruch 1 oder 2, dadurch gekennzeichnet, daß der Montageblock (8) einen über den zweiten Arm (14) der Wippe (11) ragenden Ansatz (16) mit einem Führungsloch für den Bowdenzug (15) aufweist.

4. Kniegelenk nach Anspruch 3, dadurch gekennzeichnet, daß sich der Ansatz (16) des Montageblocks (8) und der zweite Arm (14) der Wippe (11) gabelförmig und symmetrisch zur senkrecht auf der Wippachse stehenden Mittelebene des Montageblocks (8) erstrecken.

5. Kniegelenk nach einem der Ansprüche 1 bis 4, dadurch gekennzeichnet, daß das freie Ende des Bowdenzugs (15) an einem kreisförmig ausgebildeten Auslöseteil (18) anliegt und befestigt ist, das drehbar in einem am Oberschenkelteil (1) befestigten Gehäuse (19) gelagert ist.

6. Kniegelenk nach Anspruch 5, dadurch gekennzeichnet, daß das Gehäuse (19) zwei Einlaßführungen (31) für den Bowdenzug (15) aufweist, die etwa tangential auf der Seite des Auslösehebels (22) und auf der gegenüberliegenden Seite auf das Auslöseteil (18) gerichtet sind.

## Claims

1. Knee joint having a lower leg part (2) articulated on a thigh part (1) and arrested in the extended position of the knee joint, the arresting device (5) of which is arranged in the lower leg part (2) and projects into a recess (7) of the thigh part (1) with an interlocking element (6) extending upwardly beyond the contour of the lower leg part (2), the arresting being releasable by means of a Bowden drive (15) introduced into the lower leg part (2) via a rocker (11) mounted in the lower leg part (2), the first arm (10) of said rocker determining the position of the interlocking element (6) and the second arm (14) thereof being connected to the Bowden drive, characterized in that an assembly block (8) inserted into the lower leg part (2) both guides and retains a pin (6) serving as interlocking element which is urged into the arresting position by means of a spring (9) retained in the assembly block, and carries the rocker (11) at its bottom side, the first arm (10) of which is connected to the lower end of the pin (6).

2. Knee joint according to claim 1, characterized in that the assembly block (8) and the rocker (11) are joined to one another by a rocker joint (17) which is exclusively formed by a recess-web-recess and web-recess-web configuration, resp., of the adjacent sides of the assembly block (8) and the rocker (11).

3. Knee joint according to claim 1 or 2, characterized in that the assembly block (8) has a protrusion (16) extending over the second arm (14) of the rocker (11) and having a guide hole for the Bowden drive (15).

4. Knee joint according to claim 3, characterized in that the protrusion (16) of the assembly block (8) and the second arm (14) of the rocker (11) extend fork-like and symmetrical relative to the central plane of the assembly block (8) disposed vertical relative to the rocking axis.

5. Knee joint according to one of claims 1 to 4, characterized in that the free end of the Bowden drive (15) engages and is secured to a circularly formed releasing part (18) provided with a releasing lever (22), said releasing part being mounted rotatable in a housing (19) secured to the thigh part (1).

6. Knee joint according to claim 5, characterized in that the housing (19) has two inlet guides (31) for the Bowden drive (15) which are generally directed tangential on the side of the releasing lever (22) and on the opposite side to the releasing part (18).

**Revendications**

1. Articulation de genou comportant une partie jambe (2), articulée à une partie cuisse (1) et se bloquant lorsque l'articulation de genou est en position d'extension, dont le dispositif de blocage (5) est disposé dans la partie jambe (2) et vient en prise dans un évidement (7) de la partie cuisse (1) par un élément de verrouillage (6) faisant saillie vers le haut au-delà du contour de la partie jambe (1), le blocage pouvant être libéré, au moyen d'un câble Bowden (15) introduit dans la partie jambe (2), par l'intermédiaire d'un basculeur (11) logé dans la partie jambe (2), le premier bras (10) du basculeur déterminant la position de l'élément de verrouillage (6) et le second bras (14) étant relié au câble Bowden, caractérisée en ce qu'un bloc de montage (8) disposé dans la partie jambe (2) d'une part guide et maintient un loqueteau (6) servant d'élément de verrouillage qui est poussé vers la position de blocage par un ressort (9) maintenu dans le bloc de montage, et d'autre part porte sur sa face inférieure le basculeur (11) dont le premier bras (10) est relié à l'extrémité inférieure du loqueteau (6).

2. Articulation de genou conforme à la revendication 1, caractérisée en ce que le bloc de montage (8) et le basculeur (11) sont reliés l'un à l'autre par une articulation de basculement (17) exclusivement constituée par une configuration évidement-bossageévidement et respectivement bossage-évidement-bossage des côtés adjacents du bloc de montage (8) et du basculeur (11).

3. Articulation de genou conforme à l'une des revendications 1 ou 2, caractérisée en ce que le bloc de montage (8) présente un appendice (16) faisant saillie au-dessus du second bras (14) du basculeur (11) et pourvu d'un trou de guidage pour le câble Bowden (15).

4. Articulation de genou conforme à la revendication 3, caractérisée en ce que l'appendice (16) du bloc de montage (8) et le second bras (14) du basculeur (11) sont conformés en fourche, symétriquement de part et d'autre du plan médian du bloc de montage (8) pris perpendiculairement à l'axe de basculement.

5. Articulation de genou conforme à l'une des revendications 1 à 4, caractérisée en ce que l'extrémité libre du câble Bowden (15) est adjacente et fixée à un élément de déclenchement (18) de forme circulaire, lequel est pourvu d'un levier de déclenchement (22) et monté rotativement dans un boîtier (19) fixé à la partie cuisse (1).

6. Articulation de genou conforme à la revendication 5, caractérisée en ce que le boîtier (19) présente pour le câble Bowden (15) deux guidages d'entrée (31) d'orientation sensiblement tangente à l'élément de déclenchement (18) du côté du levier de déclenchement (22) et du côté opposé.

Fig. 1

1

27

28

26

29

22    30    21    24    25    18

19

32    20

23

31    31

24.

15

Fig. 2